Europäisches Patentamt

European Patent Office

Office européen des brevets

(19)

(11) Veröffentlichungsnummer: **0 177 757 B1**

(12) **EUROPÄISCHE PATENTSCHRIFT**

(45) Veröffentlichungstag der Patentschrift:
**28.12.88**

(21) Anmeldenummer: **85111164.1**

(22) Anmeldetag: **04.09.85**

(51) Int. Cl.⁴: **A 61 F 2/00,** A 61 F 2/02

(54) **Verbindung von Röhrenknochenenden.**

(30) Priorität: **07.09.84 DE 3432928**

(43) Veröffentlichungstag der Anmeldung:
**16.04.86 Patentblatt 86/16**

(45) Bekanntmachung des Hinweises auf die Patenterteilung:
**28.12.88 Patentblatt 88/52**

(84) Benannte Vertragsstaaten:
**AT BE CH FR GB IT LI LU NL SE**

(56) Entgegenhaltungen:
**AT-B- 369 257**
**US-A- 4 016 874**
**US-A- 4 453 539**
**US-A- 4 475 545**

(73) Patentinhaber: **S + G IMPLANTS GMBH,
Grapengiesserstrasse 21, D-2400 Lübeck (DE)**

(72) Erfinder: **Grundei, Hans, Gärtnergasse 4, D-2400 Lübeck
(DE)**
Erfinder: **Biehl, Thomas, Dr., Ismainger Strasse 22,
D-8000 München 80 (DE)**

(74) Vertreter: **Wilcken, Thomas, Dipl.-Ing. et al,
Musterbahn 1, D-2400 Lübeck (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt. wenn die Einspruchsgebühr entrichtet worden ist (Art. 99(1) Europäisches Patentübereinkommen).

# Beschreibung

Die Erfindung betrifft eine Vorrichtung zum Verbinden der beiden zugekehrten Enden eines durch Operation unterbrochenen Röhrenknochens oder eines Prothesenteiles mit dem Ende eines Röhrenknochens, mit einem Zapfen, dessen beide Enden in die Enden des unterbrochenen Röhrenknochens einsetzbar sind.

Bei der Behandlung von Patienten kann der Fall eintreten, dass durch Bruch eines Knochens, durch Tumore oder dergleichen ein Längenteil eines Röhrenknochens operativ entfernt und dann versucht werden muss, die verbleibenden Knochenlängen wieder miteinander zu verbinden und dabei die ursprüngliche Knochenlänge beizubehalten. Hierbei musste teilweise auf eine Schienung der verbliebenen Knochenlängen zurückgegriffen werden.

In der AT-B-369 257 ist eine einteilige längliche Gelenkprothese aus Kunststoff erwähnt, deren in die aus ausgehöhlten Knochenendabschnitte zu implantierenden Zapfenteile teilweise von Schutzhülsen umgeben sind, die drehsicher ebenfalls in die Knochenendabschnitte implantiert werden. Diese Schutzhülsen sind jedoch nur dazu gedacht, dass die eigentlichen Gelenkprothesen auch im Fall reumatischer Arthritis implantiert werden können und haben nicht die Funktion, eine wesentliche Druckbelastung in Knochenlängsrichtung zwischen zwei voneinander einen Abstand aufweisenden Knochenenden zu übertragen. Darüber hinaus sind die Schutzhülsen für eine einzige vorbestimmte Lage an der Gelenkprothese ausgebildet und daher nicht längsaxial einstellbar und feststellbar, so dass eine derartige Gesamtprothese nur für einen einzigen Anwendungsfall geeignet ist.

Die Aufgabe der Erfindung besteht in der Schaffung einer Vorrichtung der einleitend angeführten Art, mit der es möglich ist, die Enden von Mark- und Röhrenknochen, aus denen eine Teillänge operativ entfernt worden ist, unter Beibehaltung der ursprünglichen Knochenlänge auf einfache Weise wieder stabil miteinander zu verbinden.

Die Lösung dieser Aufgabe geht von der einleitend angeführten Vorrichtung aus und kennzeichnet sich dadurch, dass der Zapfen ein zylindrischer, metallischer Zapfen ist, der auf seinem Umfang mit einer Längsnut und mehreren Ringnuten versehen ist, dass die Ringnuten zwei im Abstand voneinander angeordnete Ringe mit aus den Ringnuten nach aussen vorragenden Anschlagflächen für die zugekehrten Knochenenden oder einen Ring mit einer aus der entsprechenden Ringnut nach aussen vorragenden Anschlagfläche für das Knochenende aufnehmen und dass die Längsnut zwei jeweils mit einer der Anschlagflächen verbundene, aus der Längsnut als Flächen vorragende Längsflansche bzw. einen mit der Anschlagfläche verbundenen, aus der Längsnut als Fläche vorragenden Längsflansch aufnimmt.

Durch diese Lösung kann der zylindrische Zapfen mit seinen Enden in die beiden Enden des durch die Operation unterbrochenen Markknochens eingeführt werden, wobei ein Längenteil des Zapfens, der dem Längenteil der herausgetrennten Knochenlänge entspricht, freibleibt. Dabei sind entsprechend dem freibleibenden Längenabstand in zwei der Ringnuten Anschlagflächen eingesetzt, die gegen die zugekehrten Enden des unterbrochenen Knochens zur Anlage kommen, um die Eindringtiefe des Zapfens in die Knochenhöhlen zu begrenzen, womit die ursprüngliche Knochenlänge sicher beibehalten wird. Eine Verdrehung des Zapfens ist dadurch vermieden, dass der vorstehende Längsflansch der Anschlagflächen einerseits in die Längsnut des Zapfens und andererseits durch seinen äusseren Teil in eine innere, in den jeweiligen Knochen einzufräsende Nut zum Eingriff kommt.

In bevorzugter Ausgestaltung der erfindungsgemässen Vorrichtung ist die Anschlagfläche oder sind die Anschlagflächen an geschlitzten, flachen Sicherungsringen ausgebildet, die aus den Ringnuten nach aussen vorragen und deren Umfang etwas grösser ist als der halbe Ringnutenumfang, so dass diese Sicherungsringe im Bereich der Nuten über den Zapfen geschoben werden und nach Spreizung sich wieder an den Zapfen anlegen. Diese Sicherungsringe sind in Anpassung an die herausgetrennte Knochenlänge in entsprechende Ringnuten einsetzbar, so dass die ursprüngliche Knochenlänge wiedergewonnen und beibehalten wird.

Die Erfindung wird nachstehend anhand der Zeichnung erläutert. Es zeigen:

Figur 1 eine Seitenansicht des Verbindungszapfens nach der Erfindung,

Figur 2, 3 zwei Querschnitte nach den Linien II–II und III–III der Figur 1,

Figur 4 einen Schnitt nach Linie IV–IV der Figur 1 mit in eine Ringnut eingesetztem Sicherungsring,

Figur 5 eine Seitenansicht des Sicherungsringes, gesehen in Richtung des Pfeiles X der Figur 4,

Figur 6 die gleiche Ansicht auf zwei fest miteinander verbundene Sicherungsringe.

Der Verbindungszapfen 1 ist ein metallischer zylindrischer Zapfen und vorteilhaft so ausgebaut, dass ein metallischer zylindrischer Zapfen 2 mit glattem Aussenumfang mit einem porösen oder offenzelligen Metallüberzug 3 versehen ist. Der Zapfen 1 ist möglichst in gleichen Abständen mit einer Anzahl Ringnuten 4 versehen, deren Tiefe z.B. der Dicke des Metallüberzuges 3 entspricht. Weiter ist der Zapfen 1 mit einer äusseren Längsnut 5 versehen, die die gleiche Tiefe aufweist.

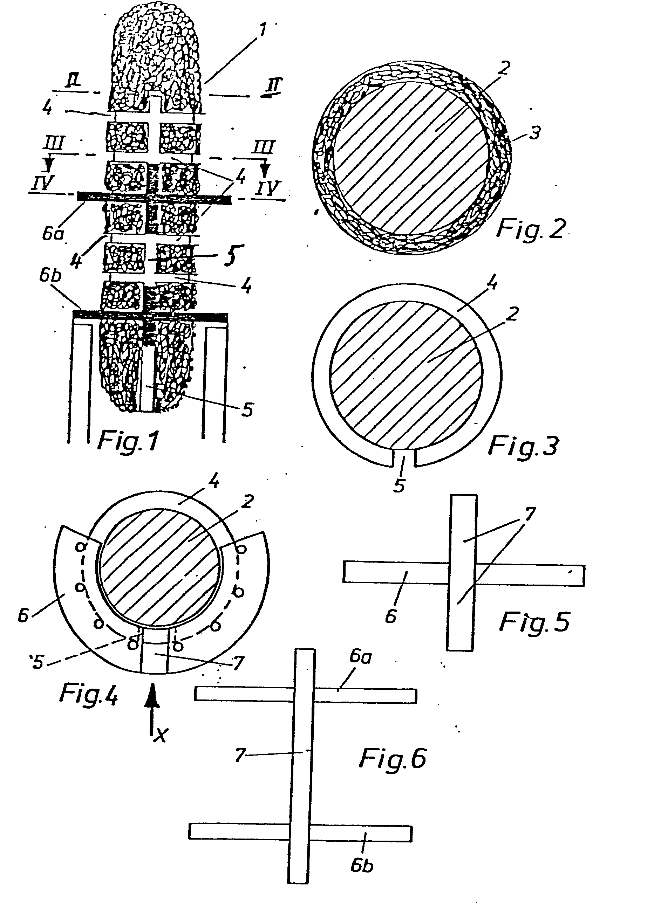

In die Ringnuten 4 ist ein flacher unterbrochener Sicherungsring 6 einsetzbar, dessen innerer Durchmesser dem Durchmesser des Zapfens 2 entspricht und dessen Umfang etwas grösser ist als der halbe Umfang des Zapfens 2. Die flachen einsetzbaren Sicherungsringe 6 ragen mit einem äusseren Flächenteil aus den Ringnuten 4 heraus, wie aus Figur 1 und 4 ersichtlich ist, und diese herausragenden Flächenteile bilden Anschläge für die Enden von Knochen, aus denen z.B. wegen eines Tumors oder eines sonstigen Schadens ein Längenstück operativ entfernt werden muss. Es wird dann das eine Zapfenende an der Knochenschnittfläche in die Markhöhle eingeführt, bis die äussere Anschlaglänge eines in eine Ringnut federnd eingesetzten Sicherungsringes 6a gegen die Knochenschnittfläche zur Anlage kommt. Entsprechend der Länge des entfernten Knochenstückes wird im Abstand vom Sicherungsring 6a ein zweiter Sicherungsring 6b in eine Ringnut 4 eingesetzt, und dann wird das hieran anschliessende Zapfenende in die Markhöhle des zweiten Knochenteiles eingesetzt bzw. eingetrieben, bis der Sicherungsring 6b gegen die Knochenschnittfläche anliegt. Damit ist die Verbindung der Enden des unterbrochenen Mark- oder Röhrenknochens hergestellt.

Um dabei eine Sicherung des Zapfens 1 gegen Verdrehung zu erreichen, ist jeder Sicherungsring 6 mit einem senkrecht zum Ring stehenden längsgerichteten Flansch 7 fest verbunden, der beim Einsetzen des Federringes 6 in die Längsnut 5 eingreift und ebenso weit nach aussen vorragt wie der Sicherungsring 6 (Figur 4). Beim Einsetzen des vorbereiteten Zapfens 1 in die Markhöhle kommt der Längsflansch 7 in eine vorher in der Markhöhle im Knochen eingefräste Nut zum Eingriff. Damit ist die Sicherung gegen Verdrehung der beiden Knochenteile zueinander gewährleistet.

Anstatt zweier getrennter Sicherungsringe 6 kann man auch zwei Sicherungsringe 6a und 6b durch einen längsgerichteten Flansch 7a (Figur 6) fest miteinander verbinden, wobei der Abstand der beiden Sicherungsringe an die zu entfernende Knochenlänge und auch an die vorhandenen Ringnuten 4 angepasst sein muss.

Wie schon erwähnt, kann der Zapfen 1 auch ein fester Teil einer Endoprothese sein, so dass dann nur ein Zapfenende bis zum Anschlag eines Sicherungsringes in die Markhöhle eines Knochens eingeführt werden muss.

**Patentansprüche**

1. Vorrichtung zum Verbinden der beiden zugekehrten Enden eines durch Operation unterbrochenen Röhrenknochens oder eines Prothesenteiles mit dem Ende eines Röhrenknochens, mit einem Zapfen (1), dessen beide Enden in die Enden des unterbrochenen Röhrenknochens einsetzbar sind, dadurch gekennzeichnet, dass der Zapfen (1) ein zylindrischer Metallzapfen ist, der auf dem Umfang mit einer Längsnut (5) und mehreren Ringnuten (4) versehen ist, dass die Ringnuten (4) zwei im Abstand voneinander angeordnete Ringe (6a, 6b) mit aus den Ringnuten nach aussen vorragenden Anschlagflächen für die zugekehrten Knochenenden oder einen Ring (6) mit einer aus der entsprechenden Ringnut nach aussen vorragenden Anschlagfläche für das Knochenende aufnehmen und dass die Längsnut (5) zwei jeweils mit einer der Anschlagflächen verbundene, aus der Längsnut als Flächen vorragende Längsflansche (7) bzw. einen mit der Anschlagfläche verbundenen, aus der Längsnut als Fläche vorragenden Längsflansch (7) aufnimmt.

2. Vorrichtung nach Anspruch 1, dadurch gekennzeichnet, dass die Tiefe der Ringnuten (4) und der Längsnut (5) der Dicke eines den Aussenumfang des zylindrischen Zapfens (1) bildenden, offenzelligen Metallbelags (3) entspricht.

3. Vorrichtung nach Anspruch 1 oder 2, dadurch gekennzeichnet, dass der oder die Anschlagflächen an geschlitzten, flachen Sicherungsringen (6) ausgebildet sind, deren Umfang etwas grösser ist als der halbe Ringnutenumfang.

4. Vorrichtung nach den Ansprüchen 1 bis 3, dadurch gekennzeichnet, dass der Längsflansch (7) in gewünschtem Abstand mit zwei in zwei Ringnuten (4) eingreifenden Sicherungsringen (6a, 6b) fest verbunden ist.

**Revendications**

1. Dispositif pour relier les deux extrémités tournées l'une vers l'autre d'un os tubulaire sectionné par une opération, ou d'une partie de prothèse avec l'extrémité d'un os tubulaire, comprenant un tourillon (1) dont les deux extrémités peuvent être insérées dans l'os tubulaire sectionné, caractérisé en ce que le tourillon (1) est un tourillon métallique cylindrique, qui est muni sur sa périphérie d'une rainure longitudinale (5) et de plusieurs rainures annulaires (4), en ce que les rainures annulaires (4) reçoivent deux anneaux (6a, 6b) disposés à une certaine distance l'un de l'autre et comprenant des surfaces de butée faisant saillie vers l'extérieur à partir des rainures annulaires et destinées aux extrémités des os qui sont tournées l'une vers l'autre, ou un anneau (6) comprenant une surface de butée pour l'extrémité de l'os et faisant saillie vers l'extérieur à partir de la rainure annulaire correspondante, et en ce que la rainure longitudinale (5) reçoit deux nervures longitudinales (7) faisant saillie sous forme de surface de la rainure longitudinale et respectivement reliées à l'une des surfaces de butée, ou une nervure longitudinale (7) faisant saillie sous forme de surface de la rainure longitudinale et reliée à la surface de butée.

2. Dispositif selon la revendication 1, caractérisé en ce que la profondeur des rainures annulaires (4) et de la rainure longitudinale (5) correspond à l'épaisseur d'une garniture métallique à cellules ouvertes (3) formant la périphérie externe du tourillon cylindrique (1).

3. Dispositif selon la revendication 1 ou 2, caractérisé en ce que la ou les surfaces de butée sont constituées sur des anneaux de fixation fendus et plats (6) dont la périphérie est sensiblement plus importante que la demi-périphérie de la rainure annulaire.

4. Dispositif selon l'une quelconque des revendications 1 à 3, caractérisé en ce que la nervure longitudinale (7) est fermement reliée à une distance désirée à deux anneaux de fixation (6a, 6b) pénétrant dans deux rainures annulaires (4).

**Claims**

1. Device for joining the two mutually opposed ends of a fistular bone severed by an operation or of a prosthetic member with the end of a fistular bone, with a pin (1) the two ends of which are insertible into the ends of the severed fistular bone, characterised in that the pin (1) is a cylindrical metal pin which is provided at the periphery with a longitudinal groove (5) and several annular grooves (4), that the annular grooves (4) receive two mutually spaced apart rings (6a, 6b) with abutment surfaces projecting outwardly from the annular grooves for the opposed bone ends, or a ring (6) with an abutment surface projecting outwards from the corresponding annular groove for the bone end, and that the longitudinal groove (5) receives two longitudinal flanges (7) each joined to one of the abutment surfaces and with surfaces projecting out of the longitudinal groove, or one longitudinal flange (7) joined to the abutment surface and with a surface projecting from the longitudinal groove.

2. Device according to Claim 1, characterised in that the depth of the annular grooves (4) and of the longitudinal groove (5) corresponds to the thickness of an open-celled metal layer (3) forming the external periphery of the cylindrical pin (1).

3. Device according to Claim 1 or 2, characterised in that the abutment surface or surfaces is or are formed on flat perforated securing rings (6) the periphery of which is somewhat greater than half the periphery of the annular grooves.

4. Device according to Claims 1 to 3, characterised in that the longitudinal flange (7) is firmly joined with required spacing to two securing rings (6a, 6b) engaging in two annular grooves (4).

1/1

Fig.1

Fig. 2

Fig.3

Fig. 5

Fig. 4

Fig.6